# EUROPEAN PATENT SPECIFICATION

(11) **EP 0 263 506 B1**
(45) Date of publication and mention of the grant of the patent: **04.08.1993**
(21) Application number: 87114623.9
(22) Date of filing: 07.10.1987
(51) Int. Cl.: A61K 31/16, A61K 37/02, A61K 31/38, A61K 31/34, C07C 233/00

(54) **Anticonvulsant composition containing amino acid derivative and use of said amino acid derivative**
Aminosäurederivate enthaltende antikonvulsive Zusammensetzungen und Verwendung dieser Aminosäurederivate
Compositions anticonvulsantes contenant des dérivés d'acides aminés et utilisation desdits dérivés d'acides aminés

(30) Priority: 07.10.1986 US 916254; 31.07.1987 US 80528
(43) Date of publication of application: 13.04.1988
(73) Proprietor: RESEARCH CORPORATION TECHNOLOGIES, INC., Tucson Arizona 85710-2815 (US)
(72) Inventor: Kohn, Harold L., Houston Texas (US); Watson, Darrell, Belton Texas (US)
(74) Representative: Brauns, Hans-Adolf, Dr. rer. nat.

(56) References cited:
- EP-A- 0 042 626
- EP-A- 0 046 707
- EP-A- 0 194 464
- BE-A- 885 303
- JOURNAL OF MEDICINAL CHEMISTRY, vol. 28, no. 5, May 1985, pages 601-606, American Chemical Society; H. KOHN et al.: "Effect of structural modification of the hydantoin ring on anticonvulsant activity"
- JOURNAL OF MEDICINAL CHEMISTRY, vol. 30, no. 3, March 1987, pages 567-574, American Chemical Society; H. KOHN et al.: "Functionalised DL-Amino acid derivatives. Potent new agents for the treatment of epilepsy

## Description

The present invention relates to compounds having central nervous system (CNS) activity which are useful in the treatment of epilepsy and other CNS disorders.

The predominant application of anticonvulsant drugs is the control and prevention of seizures associated with epilepsy or related central nervous system disorders. Epilepsy refers to many types of recurrent seizures produced by paroxysmal excessive neuronal discharges in the brain; the two main generalized seizures are petit mal, which is associated with myoclonic jerks, akinetic seizures, transient loss of consciousness, but without convulsion; and grand mal which manifests in a continuous series of seizures and convulsions with loss of consciousness.

The mainstay of treatment for such disorders has been the long-term and consistent administration of anticonvulsant drugs. Most drugs in use are weak acids that, presumably, exert their action on neurons, glial cells or both of the central nervous system. The majority of these compounds are characterized by the presence of at least one amide unit and one or more benzene rings that are present as a phenyl group or part of a cyclic system.

Much attention has been focused upon the development of anticonvulsant drugs and today many such drugs are well known. For example, the hydantoins, such as phenytoin, are useful in the control of generalized seizures and all forms of partial seizures. The oxazolidinediones, such as trimethadione and paramethadione, are used in the treatment of nonconvulsive seizures. Phenacemide, a phenylacetylurea, is one of the most well known anticonvulsants employed today, while much attention has recently been dedicated to the investigation of the diazepines and piperazines. For example, U.S. Patent Nos. US-A-4,002,764 and 4,178,378 to Allgeier, et al. disclose esterified diazepine derivatives useful in the treatment of epilepsy and other nervous disorders. U.S. Patent No. US-A-3,887,543 to Nakanishi, et al. describes a thieno [2,3-e][1,4] diazepine compound also having anticonvulsant activity and other depressant activity. U.S. Patent No. US-A-4,209,516 to Heckendorn, et al. relates to triazole derivatives which exhibit anticonvulsant activity and are useful in the treatment of epilepsy and conditions of tension and agitation. U.S. Patent No. US-A-4,372,974 to Fish, et al. discloses a pharmaceutical formulation containing an aliphatic amino acid compound in which the carboxylic acid and primary amine are separated by three or four units. Administration of these compounds in an acid pH range are useful in the treatment of convulsion disorders and also possess anxiolytic and sedative properties.

Unfortunately, despite the many available pharmacotherapeutic agents, a significant percentage of the population with epilepsy or related disorders are poorly managed. Moreover, none of the drugs presently available are capable of achieving total seizure control and most have disturbing side-effects. Clearly, current therapy has failed to "seize control" of these debilitating diseases.

The present invention relates to:
an anticonvulsant composition comprising an anticonvulsant effective amount of a compound having the following general formula I: 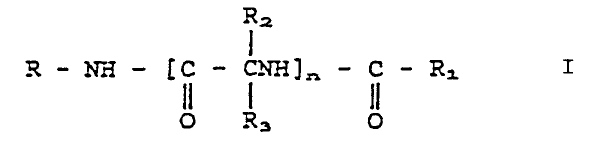
wherein R is aryl, aryl lower alkyl, heterocyclic, lower alkyl heterocyclic, polynuclear aromatic or lower alkyl polynuclear aromatic, each unsubstituted or substituted with at least one electron withdrawing substituent or at least one electron donating substituent;
R₁ is H or a lower alkyl, unsubstituted or substituted with at least one electron withdrawing substituent or at least one electron donating substituent;
R₂ and R₃, independently, are hydrogen, lower alkenyl, lower alkynyl, heterocyclic, lower alkyl heterocyclic, polynuclear aromatic, lower alkyl polynuclear aromatic, each unsubstituted or substituted with at least one electron withdrawing substituent or at least one electron donating substituent, or R₂ and R₃ can be a halogen or a heteroatom containing oxygen, nitrogen, sulfur, or phosphorous substituted with hydrogen, lower alkyl or aryl, said lower alkyl or aryl groups being substituted or unsubstituted; with the proviso that R₂ and R₃ are not both hydrogen;

n is 1 to 4;

wherein lower alkyl substituents have from 1 to 6 carbon atoms; aryl substituents have from 6 to 10 ring carbon atoms; heterocyclic substituents have up to 4 heteroatoms and up to 13 ring carbon atoms; polynuclear aromatic compounds have up to 4 fused rings and up to 18 ring carbon atoms; lower alkenyl and lower alkynyl substituents containing from 2 to 6 carbon atoms; and
wherein electron withdrawing groups can be halo, nitro, acyl, carboxyl, carboalkoxy, carboxamide, cyano, sulfonyl, sulfoxide, heterocyclic, guanidine or quaternary ammonium; and electron donating groups can be hydroxy, alkoxy, lower alkyl, amino, phenoxy, thiol, sulfide or disulfide and
a pharmaceutically acceptable carrier.

The present invention contemplates employing the compounds of Formula I in compositions of pharmaceutically acceptable dosage forms. Where the appropriate substituents are employed, the present invention also includes pharmaceutically acceptable addition salts. Moreover, the administration of an effective amount of the present compounds, in their pharmaceutically acceptable forms or the addition salts thereof, can provide an excellent regime for the treament of epilepsy, nervous anxiety, psychosis, insomnia and other related central nervous system disorders.

The alkyl groups exemplary of the substituents are lower alkyl containing from 1 to 6 carbon atoms and may be straight chain or branched. These groups include methyl, ethyl, propyl, isopropyl, butyl, isobutyl, tertiary butyl, amyl and hexyl.

The aryl groups of R, R₁, R₂ and R₃ are aromatic compounds containing from 6 to 10 ring carbon atoms; and include phenyl, α- and β-naphthyl. Moreover, the aryl groups also include organometallic compounds wherein a metal or metalloidal atom is sandwiched between two aromatic compounds, e.g., cyclopendienyl compounds. Ferrocene is an example of this latter class of compounds.

The aryl lower alkyl groups include, for example, benzyl, phenethyl, phenpropyl, phenisopropyl, phenbutyl and the like, diphenylmethyl, 1,1-diphenylethyl and 1,2-diphenylethyl.

The lower alkenyl and lower alkynyl groups contain from 2 to 6 carbon atoms and may be straight chain or branched.

Exemplary of the unsaturated alkyl substituents, i.e., lower alkenyl and lower alkynyl are vinyl, acetylenic, allyl, propenyl, butenyl, pentenyl, hexenyl, propynyl, butynl, pentynyl, hexynyl and pentadienyl.

The heterocyclic substituents contemplated by the present invention are N, O or S containing rings which may be monocyclic or bicyclic or tricyclic and which may contain up to 4 heteroatoms in the rings and which may contain up to 13 ring carbon atoms and up to a total of 18 carbon atoms. These heterocyclic substituents include heteroaromatics and saturated and partially unsaturated heterocyclic compounds such as furyl, thienyl, pyranyl, pyrrolyl, imidazoyl, pyrazolyl, pyridyl, pyrazinyl, pyrimidyl, pyridazinyl, indolyl, thiazolyl, oxazolyl, isothiazolyl, isoxazolyl, piperidyl, pyrrolinyl, piperazinyl, quinolyl, trizaolyl and tetrazolyl.

The polynuclear aromatic substituents contemplated herein are polyaromatic compounds containing up to 4 fused rings and containing up to 18 ring carbon atoms, for example, naphthyl, anthracenyl, phenanthrenyl and azulenyl.

The heteroatom containing substituents include, for example, methoxy, ethoxy, phenoxy, thiomethoxy, thioethoxy, thiophenoxy, methylamino, ethylamino, anilino, dimethylamino, trimethylamino, fluoro, chloro, bromo and iodo.

The aryl groups such as phenyl, ferrocenyl, the alkyl groups, the aryl lower alkyl groups, the lower alkenyl group, the lower alkynyl groups and heterocyclic, lower alkyl heterocyclic, polynuclear aromatic, and lower alkyl polynuclear aromatic may carry one or more substituents which can be characterized as either electron withdrawing groups such as halo, including bromo, fluoro, chloro, iodo, nitro, acyl, carboxyl, carboalkoxy, carboxamide, cyano, sulfonyl, sulfoxide, heterocyclic, guanidine and quaternary ammonium, or as electron donating groups such as hydroxy, alkoxy including methoxy, ethoxy, lower alkyl, amino, phenoxy, thiol, sulfide and disulfide. One skilled in the art will appreciate that the aforesaid substituents may have electron donating or electron withdrawing properties under different chemical conditions. Moreover, the present invention contemplates any combination of substituents selected from the above-defined groups.

Preferred compounds of the present invention have the following general formula: 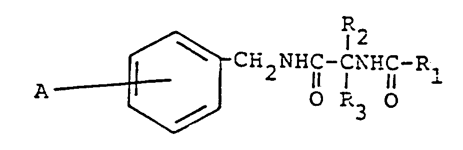
wherein R₁ is H or lower alkyl, R₂ and R₃ are as defined above and A is one to three substituents selected from the above-defined groups.

The alkyl groups of R₁ can be unsubstituted or substituted with one or more substituents which can be characterized as either electron withdrawing groups or electron donating groups as defined above.

The alkyl groups of R₂ and R₃, including the alkyl portion of the aryl alkyl, or the alkyl heterocyclic and alkyl polynuclear aromatic groups, or the alkyl or aryl groups of the heteroatom containing substituents, as well as the alkenyl, alkynyl, aryl, heterocyclic and polynuclear aromatic groups of R₂ and R₃, may also be unsubstituted or substituted with one or more substituents which can be characterized as either electron withdrawing groups or electron donating groups as defined above.

The preferred compounds of the present invention are those where n is 1 but di-, tri- and tetra-peptides are acceptable.

The compounds of the present invention may contain one (1) or more asymmetric carbon atoms and may exist in racemic and optically active forms. Depending upon the substituents, the present compounds may form addition salts as well. All of these forms are contemplated to be within the scope of this invention including mixtures of the stereoisomeric forms.

The following three schemes of preparation are generally exemplary of the process which can be employed for the preparation of the present complex: 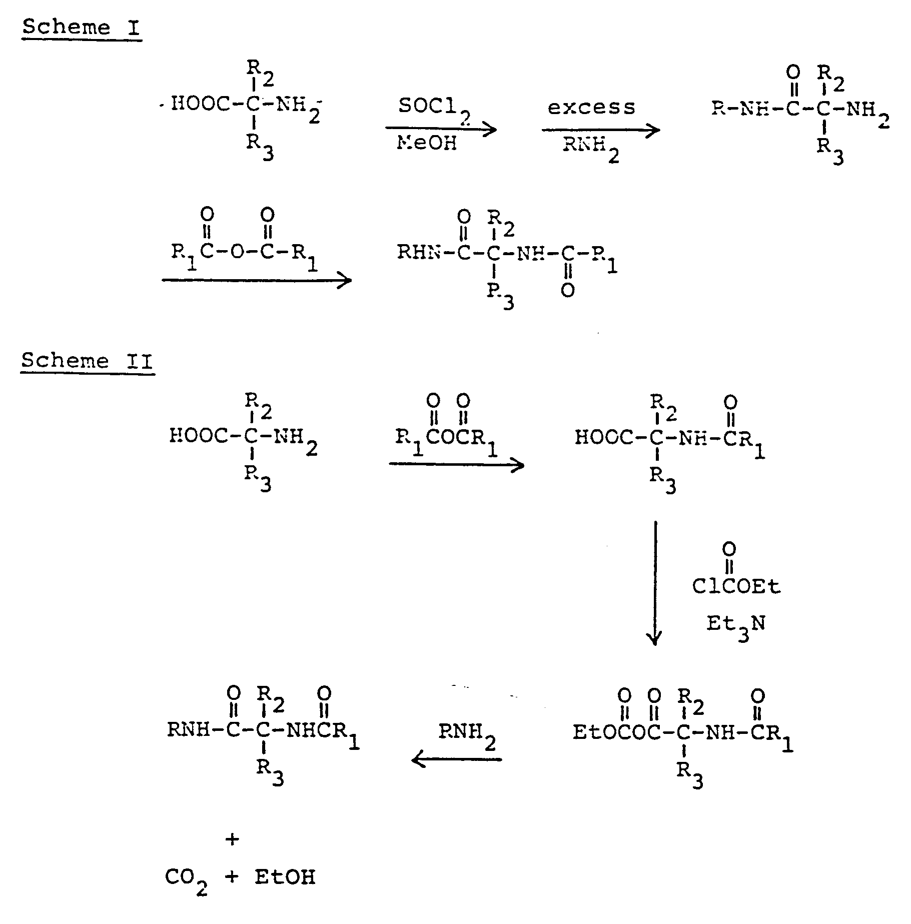 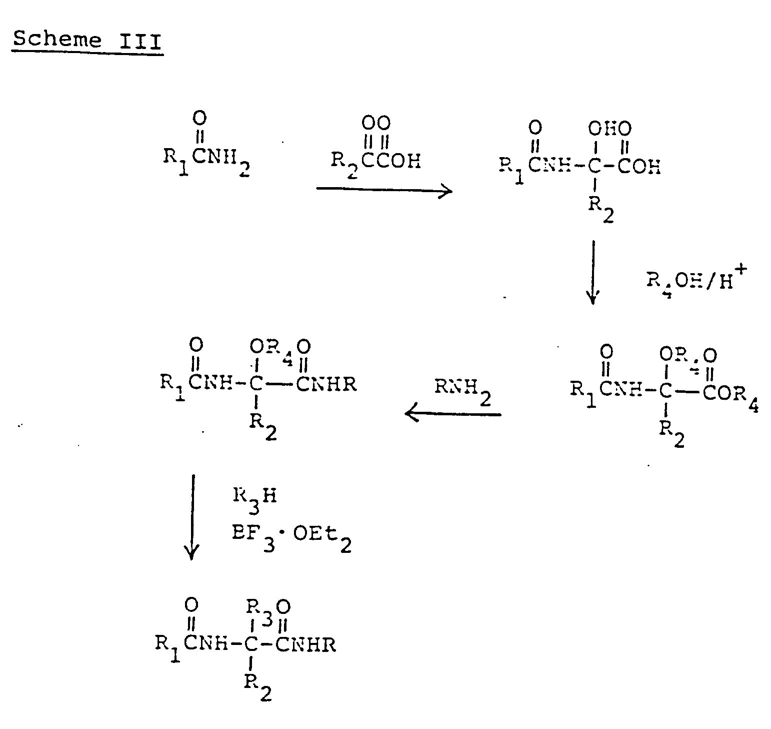
More specifically, these compounds can be prepared by art-recognized procedures from known compounds or readily preparable intermediates. For instance, compounds of Formula I can be prepared by reacting amines of Formula II with an acylating derivative of a carboxylic acid of Formula III under amide forming conditions: 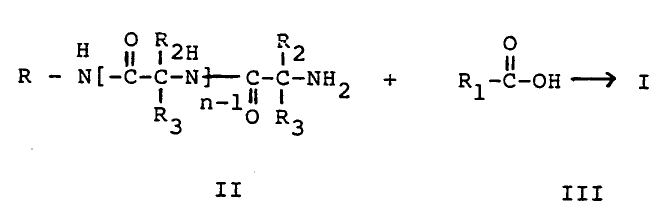
wherein R, R₁, R₂, R₃ are as defined hereinabove.

Alternatively, the compound of Formula I can be prepared by reacting an amine of Formula IV with an acylating derivative of a carboxylic acid of Formula V under amide forming conditions: 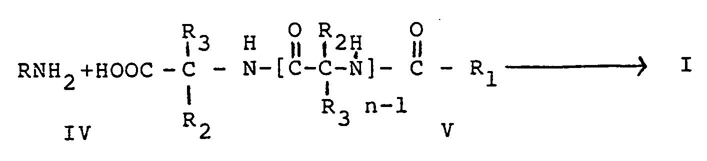
wherein R, R₁, R₂ and R₃ are as defined hereinabove.

Another useful method for preparing a compound of Formula I involves simple substitution reactions.

An exemplary procedure is as follows: 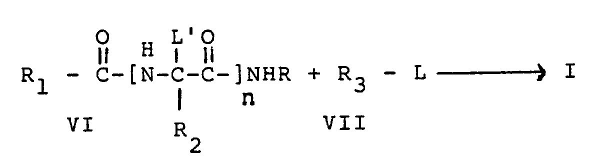
wherein R, R₁, R₂ R₄ and n have the aforesaid meanings and R₃ is defined heretofore except it is not aryl, heteroaromatic or polynuclear aromatic and L and L' are independently a good leaving group, such as halide, tosylates, mesoylates, brosylates, benzyloxy and the like. In this procedure the amine of Formula VI is reacted with a compound of Formula VII under substitution conditions. The reaction may take place in the presence of an acid, such as inorganic acid, e.g., hydrochloric acid, sulfuric acid or Lewis acid, such as boron trifluoride and the like or in the presence of a base, such as triethylamine.

However, when R₃ is heteroaromatic, aryl or polynuclear aromatic, L is hydrogen. In the procedure under these circumstances, the reaction should take place in the presence of an acid catalyst, such as an inorganic acid, e.g., hydrochloric acid or a Lewis acid, such as borontrifluoride.

The amide forming conditions referred to herein involve the use of known derivatives of the described acids, such as the acylhalides, (e.g., 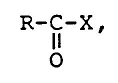
wherein X is Cl, Br, and the like), anhydrides (e.g., 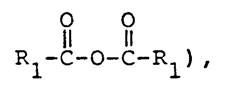
mixed anhydrides, lower alkyl esters, carbodiimides, carbonyldiimidazoles, and the like. It is preferred that the acylating derivative used is the anhydride;

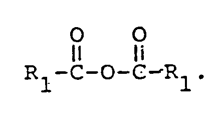

As in any organic reaction, solvents can be employed such as methanol, ethanol, propanol, acetone, tetrahydrofuran, dioxane, dimethylformamide, dichloromethane and chloroform. The reaction is normally effected at or near room temperature, although temperatures from 0°C up to the reflux temperature of the reaction mixture can be employed.

As a further convenience, the amide forming reaction can be effected in the presence of a base, such as tertiary organic amine, e.g., triethylamine, pyridine and picolines, particularly where hydrogen halide is formed by the amide forming reaction, e.g., acyl halide and the amine of Formula II. Of course, in those reactions where hydrogen halide is produced, any of the commonly used hydrogen halide acceptors can also be used.

The exact mineral acid or Lewis acid employed in the reaction will vary depending on the given transformation, the temperature required for the conversion and the sensitivity of the reagent toward the acid in the reaction employed.

The various substituents on the present compounds, e.g., as defined in R, R₁, R₂ and R₃ can be present in the starting compounds, added to any one of the intermediates or added after formation of the final products by the known methods of substitution or conversion reactions. For example, the nitro groups can be added to the aromatic ring by nitration and the nitro group converted to other groups, such as amino by reduction, and halo by diazotization of the amino group and replacement of the diazo group. Alkanoyl groups can be substituted onto the aryl groups by Friedel-Crafts acylation. The acyl groups can be then transformed to the corresponding alkyl groups by various methods, including the Wolff-Kishner reduction and Clemmenson reduction. Amino groups can be alkylated to form mono, dialkylamino and trialkylamino groups; and mercapto and hydroxy groups can be alkylated to form corresponding thioethers or ethers, respectively. Primary alcohols can be oxidized by oxidizing agents known in the art to form carboxylic acids or aldehydes, and secondary alcohols can be oxidized to form ketones. Thus, substitution or alteration reactions can be employed to provide a variety of substituents throughout the molecule of the starting material, intermediates, or the final product.

In the above reactions, if the substituents themselves are reactive, then the substituents can themselves be protected according to the techniques known in the art. A variety of protecting groups known in the art may be employed. Examples of many of these possible groups may be found in "Protective Groups in Organic Synthesis," by T.W. Green, John Wiley & Sons, 1981.

The present compounds obviously exist in stereoisomeric forms and the products obtained thus can be mixtures of the isomers, which can be resolved. Alternatively, by selection of specific isomers as starting compounds or synthetic intermediates, the preferred stereoisomer can be produced.

The active ingredients of the therapeutic compositions and the compounds of the present invention exhibit excellent anticonvulsant activity when administered in amounts ranging from 10 mg to 100 mg per kilogram of body weight per day. A preferred dosage regimen for optimum results would be from 20 mg to 50 mg per kilogram of body weight per day, and such dosage units are employed that a total of from 1.0 gram to 3.0 grams of the active compound for a subject of about 70 kg of body weight are administered in a 24-hour period. This dosage regimen may be adjusted to provide the optimum therapeutic response and is preferably administered one to three times a day in dosages of about 600 mg per administration. For example, several divided doses may be administered daily or the dose may be proportionally reduced as indicated by the exigencies of the therapeutic situation. A decided practical advantage is that the active compound may be administered in an convenient manner such as by the oral, intraveneous (where water soluble), intramuscular or subcutaneous routes.

The active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups and wafers. Such compositions and preparations should contain at least 1% of active compound. The percentage of the compositions and preparations may, of course, be varied and may conveniently be between 5 to 80% of the weight of the unit. The amount of active compound in such therapeutically useful compositions is such that a suitable dosage will be obtained. Preferred compositions or preparations according to the present invention are prepared so that an oral dosage unit form contains between 5 and 1000 mg of active compound.

The tablets, troches, pills, capsules and the like may also contain the following: A binder such as gum tragacanth, acacia, corn starch or gelatin; excipients such as dicalcium phosphate; a disintegrating agent such as corn starch, potato starch and alginic acid; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions (where water soluble) or dispersions and sterile powders for the extemporaneous preparation of sterile injectable solutions or dispersions. In all cases the form must be sterile and must be fluid to the extent that easy springability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol,), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and the freeze-drying technique which yield a powder of the active ingredient plus any additional desired ingredient from previously sterile-filtered solution thereof.

As used herein, "pharmaceutically acceptable carrier" includes any and all solvents, dispersion media, coatings, antibacterial and antifungal agents, isotonic and absorption delaying agents. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.

It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active material calculated to produce the desired therapeutic effect in association with the required pharmaceutical carrier. The specification for the novel dosage unit forms of the invention are dictated by and directly dependent on (a) the unique characteristics of the active material and the particular therapeutic effect to be achieved, and (b) the limitations inherent in the art of compounding such an active material for the treatment of disease in living subjects having a diseased condition in which bodily health is impaired as herein disclosed in detail.

The principal active ingredient is compounded for convenient and effective administration in effective amounts with a suitable pharmaceutically acceptable carrier in dosage unit form as hereinbefore disclosed. A unit dosage form can, for example, contain the principal active compound in amounts ranging from 5 to 1000 mg, with from 250 to 750 mg being preferred. Expressed in proportions, the active compound is generally present in from 10 to 750 mg/ml of carrier. In the case of compositions containing supplementary active ingredients, the dosages are determined by reference to the usual dose and manner of administration of the said ingredients.

For a better understanding of the present invention together with other and further objects, reference is made to the following description and example.

### EXAMPLE I

General Methods. Melting points were determined with a Thomas-Hoover melting point apparatus and are uncorrected. Infrared spectra (IR) were run on a Beckman IR-4250 and Perkin-Elmer 1330 spectrophotometer and calibrated against the 1601-cm⁻¹ band of polystyrene. Absorption values are expressed in wave numbers (cm⁻¹). Proton nuclear magnetic resonance (¹H NMR) spectra were recorded on Varian Associates Models T-60 and FT-80A, and Nicolet NT-300 NMR spectrometers. Carbon nuclear magnetic resonance (¹³C NMR) spectra were run on a Varian Associates Models FT-80A and Nicolet NT-300 instrument. Chemical shifts are in parts per million (δ values) relative to Me₄Si, and coupling constants (J values) are in hertz. Mass spectral data were obtained at an ionizing voltage of 70 eV on a Hewlett-Packard 5930 gas chromatograph-mass spectrometer and a Bell-Howell 21-491 spectrometer. High-resolution (EI mode) mass spectra were performed by Drs. James Hudson and John Chinn at the Department of Chemistry, University of Texas at Austin, on a CEC21-110B double-focusing magnetic-sector spectrometer at 70 eV. Elemental analyses were obtained at Spang Microanalytical Laboratories, Eagle Harbor, MI.

The solvents and reactants were of the best commercial grade available and were used without further purification unless noted. All anhydrous reactions were run under nitrogen, and all glassware was dried before use. In particular, acetonitrile and triethylamine were distilled from CaH₂, while dichloromethane was distilled from P₂O₅. Acetic anhydride, benzaldehyde and ethyl chloroformate were fractionally distilled.

### Preparation of N-Acetyl-D- and L-amino acid-N-benzylamides.

General Procedure. The D- or L-amino acid amide (11 mmol) was dissolved in dichloromethane (15 mL) and then acetic anhydride (1.23 g, 1.40 mL, 12 mmol) was added dropwise. The solution was stirred at room temperature (18 h) and then concentrated to dryness. The residue was recrystallized from chloroform/hexane.

### Preparation of N-Acetyl-D,L-methionine-N-benzylamide.

N-Acetyl-D,L-methionine (4.78 g, 25 mmol) was combined with acetonitrile (75 mL) and the mixture was placed into an ice/salt water bath (-5°C). Triethylamine (2.53 g, 3.48 mL, 25 mmol) was added dropwise, followed by ethyl chloroformate (2.71 g, 2.39 mL, 25 mmol). All additions were done slowly so that the temperature of the mixture did not rise above 0°C. The mixture was then stirred at -5°C (20 min). Benzylamine (3.00 g, 3.06 mL, 28 mmol) in acetonitrile (5 mL) was added dropwise and the mixture was stirred at -5°C (1 h) and then room temperature (18 h).

The mixture was filtered and a white precipitate was collected and dried in vacuo and identified as the desired product (¹H NMR and ¹³C NMR analyses). The filtrate was concentrated in vacuo and the residue was combined with hot tetrahydrofuran (50 mL) and cooled in the freezer (3 h), resulting in the formation of a white precipitate. The mixture was filtered and the precipitate was collected, dried in vacuo, and identified as triethylammonium hydrochloride.

The latter filtrate containing tetrahydrofuran was concentrated in vacuo and the resulting residue was purified by flash column chromatography (ethyl acetate). A white solid (R_{f} = 0.50, ethyl acetate) was isolated and was identified as the desired product (¹H NMR and ¹³C NMR analyses). The two solids identified as N-acetyl-D,L-methionine-N-benzylamide were combined and recrystallized from benzene/petroleum ether (30-60°C).
Yield: 2.98 g (43%).
mp 134-135°C.
¹H NMR (DMSO-d₆): δ 1.69-1.94 (m, 2H), 1.87 (s, 3H), 2.02 (s, 3H), 2.29-2.59 (m, 2H), 4.10-4.53 (m, 1H), 4.29 (d,J = 6.0 Hz, 2H), 7.26 (s, 5H), 8.12 (d,J = 8.5 Hz, 1H), 8.47 (t,J = 6.0 Hz, 1H).
¹³C NMR (DMSO-d₆): 14.6, 22.5, 29.7, 31.8, 42.0, 52.0, 126.6, 127.0 (2C), 128.2 (2C), 139.4, 169.5, 171.4 ppm.
IR (KBr): 3280, 1630, 1545, 1460, 750, 700 cm⁻¹.
Mass spectrum, m/e (relative intensity): 280 (3), 206 (100), 164 (29), 146 (20), 106 (54), 91 (76), 77 (14), 65 (24).

| Elemental analysis | | | |
|---|---|---|---|
| Calculated for C₁₄H₂₀N₂O₂S | 59.96% C; | 7.20% H; | 9.99% N. |
| Found | 60.02% C; | 7.14% H; | 9.91% N. |

### Preparation of D,L-α-Acetamido-N-benzyl-3-thiopheneacetamide.

D,L-α-Acetamido-3-thiopheneacetic acid (2.99 g, 15 mmol) was combined with acetonitrile (60 mL) and the mixture was placed into an ice/salt water bath (-5°C). Triethylamine (1.51 g, 2.10 mL, 15 mmol) was added dropwise, followed by ethyl chloroformate (1.63 g, 1.43 mL, 15 mmol). All additions were done slowly so that the temperature of the mixture did not rise above 0°C. The mxture was then stirred at -5°C (20 min). Benzylamine (1.77 g, 1.80 mL, 16.5 mmol) in acetonitrile (10 mL) was added dropwise and the mixture was stirred at -5°C (1 h) and then room temperature (18 h). The mixture was concentrated in vacuo and the residue was combined with hot tetrahydrofuran (50 mL) and cooled in the freezer (3 h), resulting in the formation of a white precipitate. The mixture was filtered and the precipitate was collected, dried in vacuo, and identified as triethylammonium hydrochloride (¹H NMR analysis). The filtrate was concentrated in vacuo and the resulting yellow colid was recrystallized from 1:1 95% ethanol/water.
Yield: 1.91 g (44%).
mp 198-199°C.
¹H NMR (DMSO-d₆): δ 1.91 (s, 3H), 4.29 (d,J = 5.2 Hz, 2H), 5.61 (d,J = 7.9 Hz, 1H), 7.15-7.50 (m, 3H), 8.55 (d,J = 7.9 Hz, 1H), 8.74 (t,J = 5.2 Hz, 1H).
¹³C NMR (DMSO-d₆): 22.3, 42.0, 52.5, 122.4, 126.1, 126.7, 127.0 (3C), 128.2 (2C), 139.0, 139.2, 169.0, 169.8 ppm.
IR (KBr): 3460, 1675, 1570, 1400, 720, 695 cm⁻¹.
Mass spectrum, m/e (relative intensity): 288 (2), 245 (3), 155 (88), 112 (100), 91 (31), 85 (17), 65 (7).

| Elemental analysis | | | |
|---|---|---|---|
| Calculated for C₁₅H₁₆N₂O₂S | 62.48% C; | 5.59% H; | 9.71% N. |
| Found | 62.41% C; | 5.47% H; | 9.55% N. |

### Preparation of D,L-α-Acetamido-N-benzyl-2-thiopheneacetamide

N-Acetyl-D,L-ethoxyglycine-N-benzylamide (6.26 g, 25 mmol) was combined with dry ether (175 mL) and then boron trifluoride etherate (5.68 g. 5.0 mL, 40 mmol) was added dropwise, resulting in a homogeneous solution. After stirring a short time, a small amount of a yellow oil separated from the solution. Thiophene (8.41 g, 8.0 mL, 100 mmol) was then added dropwise via syringe and the reaction was stirred at room temperature (4 d). The mixture was cooled in an ice bath and cold aqueous saturated NaHCO₃ (200 mL) was added and the aqueous layer was extracted with ethyl acetate (2 x 100 mL). The organic washings and the original ether layer were combined, dried (Na₂SO₄), and concentrated in vacuo. The residue was purified by flash column chromatography, using 94:6 chloroform/methanol as an eluant (R_{f} = 0.7 94:6 chloroform/methanol), and then recrystallized from benzene.
Yield: 2.67 g (37%).
mp 167-169°C.
¹H NMR (DMSO-d₆): δ 1.91 (s, 3H), 4.31 (d,J = 6.0 Hz, 2H), 5.74 (d,J = 7.9 Hz, 1H), 6.99-7.44 (m, 8H), 8.64 (d,J = 7.9 Hz, 1H), 8.85 (t,J = 6.0 Hz, 1H).
¹³C NNR (DMSO-d₆): 22.4, 42.3, 52.2, 125.6, 125.8, 126.6, 126.9, 127.3 (2C), 128.3 (2C), 139.0, 141.4, 169.2, 169.3 ppm.
Mass spectrum, m/e (relative intensity): 289 (2), 181 (6), 155 (100), 112 (100), 91 (100), 85 (34), 74 (24).

| Elemental analysis | | | |
|---|---|---|---|
| Calculated for C₁₅H₁₆N₂O₂S | 62.48% C; | 5.59% H; | 9.71% N. |
| Found | 62.64% C; | 5.73% H; | 9.61% N. |

### Preparation of D,L-α-Acetamido-N-benzyl-2-furanacetamide.

N-Acetyl-D,L-2-(2-furyl)glycine (0.47 g, 2.56 mmol) was combined with acetonitrile (10 mL) and cooled to -5°C (ice/salt water bath). Triethylamine (0.26 g, 0.36 mL, 2.56 mmol) was then rapidly added and the mixture stirred at -5°C (3 min). Ethyl chloroformate (0.28 g, 0.25 mL, 2.56 mmol) was added dropwise beween -4°C and -3°C, and the resulting suspension was stirred at -4°C (20 min), and then an acetonitrile solution (2 mL) of benzylamine (0.30 g, 0.31 mL, 2.82 mmol) was carefully added. During the addition of benzylamine the temperature of the solution did not go above 0°C. The mixture was stirred at -5°C (1 h) and at room temperature (18 h), and then concentrated in vacuo. The residue was then triturated with hot tetrahydrofuran (5 mL), cooled at -16°C (3 h), and the resulting white precipitate was filtered and identified as triethylamine hydrochloride (¹H NMR, 60 MHz, δ 1.00 (t,J = 7.5 Hz, CH₃), 2.82 (q,J = 7.5 Hz, CH₂), 3.83 (s, NH)). The filtrate was evaporated to dryness in vacuo and the resulting oil purified by flash chromatography (98:2 chloroform/methanol) to give 0.09 g (13%) of the desired product as a white solid: R_{f} 0.30 (98:2 chloroform/methanol).
mp 178-179°C.
¹H NMR (300 MHz, DMSO-d₆): δ 1.90 (s, CH₃), 4.31 (d,J = 6.0 Hz, CH₂), 5.58 (d,J = 8.1 Hz, CH), 6.27-6.33 (m, C₃^{.}H), 6.40-6.44 (m, C₄^{.}H), 7.20-7.36 (m, Ph), 7.60-7.64 (m, C₅^{.}H), 8.57 (d,J = 8.1 Hz, NH), 8.73 (t,J = 6.0 Hz, NH).
¹³C NMR (300 MHz, DMSO-d₆): 22.35 (CH₃), 42.27 (CH₂), 50.95 (CH), 107.60 (C₃^{.}), 110.55 (C₄^{.}), 126.82 (2C₂^{..} or 2C₃^{..}), 127.08 (2C₂^{..} or 2C₃^{..}), 128.27 (C₄^{..}), 139.05 (C₁^{..}), 142.58 (C₅^{.}),
151.16 (C₂^{.}), 168.02 (CH₃CO), 169.30 (NHCO) ppm.
IR (KBr): 3230, 1625 (br), 1525 (br), 1375, (br), 1230, 1090, 690 cm⁻¹.
Mass spectrum, m/e (relative intensity) : 273 (1), 139 (100), 96 (94), 91 (51), 65 (9).

| Elemental analysis | | | |
|---|---|---|---|
| Calculated for C₁₅H₁₆N₂O₃ | 66.16% C; | 5.83% H; | 10.29% N. |
| Found | 65.92% C; | 5.83% H; | 10.15% N. |

### Preparation of D,L-α-Acetamido-N-benzyl-2-pyrroleacetamide.

2-Acetamido-N-benzyl-2-ethoxyacetamide (2.00 g, 8.0 mmol) was suspended in anhydrous ethyl ether (60 mL), and then boron trifluoride etherate (1.82 g, 1.57 mL, 12.8 mmol) was added in one portion and the resulting solution was stirred (15 min). The pyrrole (2.14 g, 2.22 mL, 32 mmol) was then added in one portion and the solution was stirred at room temperature (48 h) during which time a precipitate formed. Hexanes (80 mL) were then added to the suspension, and the mixture was filtered and the brown semi-solid was triturated with 95:5 chloroform/methanol (30 mL) to furnish a green solid. This material was purified by flash chromatography (95:5 chloroform/methanol) to yield 0.94 g (35%) of the desired product as a white solid: R_{f} 0.29 (96:4 chloroform/methanol).
mp 174-175°C.
¹H NMR (300 MHz, CD₃CN) : δ 1.93 (s, CH₃), 4.35 (d,J = 6.0 Hz, CH₂), 5.42 (d,J = 6.9 Hz, CH), 6.00-6.18 (m, C₃^{.}H, C₄^{.}H), 6.68-6.72 (m, C₅^{.}H), 7.04 (d,J = 6.9 Hz, NH), 7.17 (t,J = 6.0 Hz, NH), 7.10-7.47 (m, Ph), 9.10-9.80 (br s, NH).
¹³C NMR (300 MHz, CD₃CN): 23.02 (CH₃), 43.83 (CH₂), 52.65 (CH), 107.57 (C₃^{.}), 108.85 (C₄^{.}), 119.33 (C₅^{.}), 127.96 (C₂^{.}), 128.01 (2C₂^{..} or 2C₃^{..}), 128.09 (2C₂^{..} or 2C₃^{..}), 129.49 (C₄^{..}), 140.01 (C₁^{..}), 170.94 (COCH₃), 171.21 (CONH) ppm.
IR (KBr): 3320, 1570 (br), 1470 (br), 1330, 1230, 950, 890, 860, 760, 710, 690, 655 cm⁻¹.
Mass spectrum, m/e (relative intensity): 171 (12), 228 (2), 213 (1), 180 (2), 164 (9), 137 (93), 108 (20), 95 (100), 91 (38), 82 (35), 68 (15).

| High resolution mass spectral analysis | |
|---|---|
| Calculated for C₁₅H₁₇N₃O₂ | 271.13208. |
| Found | 271.13144. |

### Preparation of D,L-2-Acetamido-N-benzyl-2-ethoxyacetamide.

An ethanolic solution (420 mL) of ethyl 2-acetamido-2-ethoxyacetate (27.92 g, 147 mmol) and benzylamine (23.70 g, 24 mL, 221 mmol) was stirred at 40-45°C for 3 days. The reaction mixture was evaporated in vacuo and the residue recrystallized (1:3:5 tetrahydrofuran/hexanes (650 mL)) to yield 25.80 g (70%) of the desired product as beige crystals: R_{f} 0.59 (95:5 chloroform/methanol).
mp 153-155°C.
¹H NMR (300 MHz, CDCl₃): δ 1.20 (t,J = 7.0 Hz, CH₃), 2.07 (s, CH₃), 3.60-3.76 (m, CH₂CH₃), 4.40-4.54 (m, CH₂NH), 5.60 (d,J = 8.7 Hz, CH), 6.63 (d,J = 8.7 Hz, NH), 7.00 (br s, NH), 7.26-7.36 (m, Ph).
¹³C NMR (300 MHz, CDCl₃): 15.06 (CH₃CH₂), 23.25 (CH₃CO), 43.60 (CH₂NH), 64.51 (CH₂CH₃), 77.43 (CH), 127.69 (2C₂^{..} or 2C₃^{..}, C₄^{..}), 128.79 (2C₂^{..} or 2C₃^{..}), 137.57 (C₁^{..}), 168.13 (COCH₃), 171.29 (CONH) ppm.
IR (KBr): 3260, 1630 (br), 1550 (sh), 1505 (br), 1380, 1360, 1230, 1115, 1060, 1015, 890, 745, 690 cm⁻¹.
Mass spectrum, m/e (relative intensity): 251 (4), 163 (9), 116 (98), 106 (34), 91 (98), 74 (100).

| Elemental analysis | | | |
|---|---|---|---|
| Calculated for C₁₃H₁₈N₂O₃ | 62.38% C; | 7.25% H; | 11.19% N. |
| Found | 62.49% C; | 7.27% H; | 11.24% N. |

### Preparation of D,L-2-Acetamido-N-benzyl-2-methoxyacetamide.

To a methanolic solution (180 mL) of methyl 2-acetamido-2-methoxyacetate (8.73 g, 54 mmol) was rapidly added benzylamine (8.68 g, 8.80 mL, 81 mmol) and then the mixture was stirred at 50°C (3 days) during which time a beige precipitate appeared. The solvent was removed in vacuo and the resulting precipitate was recrystallized from tetrahydrofuran (2x) to give 7.67 g (32%) of the desired product as beige crystals: R_{f} 0.35 (95:5 chloroform/methanol).
mp 145-146°C.
¹H NMR (300 MHz, CDCl₃): δ 2.06 (s, CH₃CO), 3.37 (s, CH₃O), 4.40-4.35 (m, CH₂), 5.52 (d,J = 8.7 Hz, CH), 7.12 (d,J = 8.7 Hz, NH), 7.20-7.40 (m, Ph, NH).
¹³C NMR (300 MHz, CDCl₃): 23.03 (CH₃CO), 43.51 (CH₂), 55.84 (CH₃O), 78.94 (CH), 127.62 (C₄^{..}), 127.70 (2C₂^{..} or 2C₃^{..}), 128.70 (2C₂^{..} or 2C₃^{..}), 137.45 (C₁^{..}), 166.91 (COCH₃), 171.57 (CONH) ppm.
IR (KBr): 1260, 1825 (br), 1550, 1505, 1435, 1390, 1370, 1230, 1120, 1050, 935, 890, 690 cm⁻¹.
Mass spectrum, m/e (relative intensity): 237 (1), 205(2), 177 (2), 163 (4), 146 (1), 134 (1), 121 (2), 106 (26), 102 (98), 91 (95), 77 (13), 61 (100).

| Elemental analysis | | | |
|---|---|---|---|
| Calculated for C₁₂H₁₆N₂O₃ | 61.00% C; | 6.83% H; | 11.86% N. |
| | 60.91% C; | 6.85% H; | 11.66% N. |

in the eye prior to application of the electrodes so as to prevent the death of the animal. Protection in this test was defined as the abolition of the hind limb tonic extension component of the seizure. The Subcutaneous Pentylenetetrazole (Metrazol^{R}) Seizure Threshold Test (sc Met) entailed the administration of 85 mg/kg of pentylenetetrazole as a 0.5% solution subcutaneously in the posterior midline. This amount of pentylenetetrazole was expected to produce seizures in greater than 95% of mice. The animal was observed for 30 minutes. Protection was defined as the failure to observe even a threshold seizure (a single episode of clonic spasms of at least 5 sec duration). The effects of the compounds on forced and spontaneous motor activity were evaluated in mice using the Rotorod Test (Tox) or, in some cases, as indicated herein, a horizontal screen assay (HS). The animal was placed on a one-inch diameter knurled plastic rod rotating at 6 rpm after the administration of the drug. Normal mice can remain on a rod rotating at this speed indefinitely. Neurologic toxicity was defined as the failure of the animal to remain on the rod for one minute. The MES and so Met Tests were conducted on single animals while four mice were utilized for the Tox Test. The dose effect behavior of the compounds was evaluated using the above-described procedures by the administration of varying dose levels, treating normally eight mice at each dose. Table I includes an evaluation of the Median Effective Dose (ED50) and the Median Toxic Dose (TD50) of representative compounds. Mice were tested with varying doses of the anticonvulsant to define the limits of complete protection (or toxicity) and no protection (or no toxicity), as well as three points in between these limits. The Median Effective Dose (ED50) was defined as the dose which produced the
Pharmacology. The following compounds were tested for anticonvulsant activity using male Carworth Farms #1 mice:
N-Acetyl-D,L-methionine-N-benzylamide
D,L-α-Acetamido-N-benzyl-3-thiopheneacetamide
D,L-α Acetamido-N-benzyl-2-thiopheneacetamide
D,L-α-Acetamido-N-benzyl-2-furanacetamide
D,L-α-Acetamido-N-benzyl-2-pyrroleacetamide
D,L-2-Acetamido-N-benzyl-2-ethoxyacetamide
D,L-2-Acetamido-N-benzyl-2-methoxyacetamide
The compound was given in three dose levels (30, 100, 300 mg) and subsequently compared with phenytoin, phenobarbital, mephenytoin and phenacemide (see Table I). N-Acetyl-D,L-alanine-N'-benzylamide was tested at 600 mg/mL as well. Seizures were then artificially induced by either electroshock or pentylenetetrazole. Maximal electroshock seizures (MES) were elicited with a 60 cycle alternating current of 50 mA intensity (5-7 times that necessary to elicit minimal electroschock seizures) delivered for 0.2 sec via corneal electrodes. A drop of 0.9% saline was instilled desired endpoint in 50% of the animals. The Median Toxicity Dose (TD50) was the dose which elicited evidence of minimal neurological toxicity in 50% of the animals.

D,L-α-acetamido-N-benzyl-2-furanacetamide, D,L-α-acetamido-N-benzyl-2-pyrroleacetamide exhibited anticonvulsant activity at doses less than 33 mg/kg. As indicated in Table I, D,L-α-acetamido-N-benzyl-2-thiopheneacetamide, D,L-α-acetamido-N-benzyl-2-furanacetamide, D,L-α-acetamido-N-benzyl-2-pyrroleacetamide and D,L-2-acetamido-N-benzyl-2-ethoxyacetamide, possessed ED50 values in the MES test that compared well with phenobarbital, while their TD50s were of similar magnitude or considerably higher. The TD50 of D,L-α-acetamido-N-benzyl-3-thiopheneacetamide, D,L-α-acetamido-N-benzyl-2-thiopheneacetamide, D,L-α-acetamido-N-benzyl-2-furanacetamide, D,L-α-acetamido-N-benzyl-2-pyrroleacetamide, D,L-2-acetamido-N-benzyl-2-ethoxyacetamide and D,L-2-acetamido-N-benzyl-2-methoxyacetamide were evaluated using a horizontal screen assay (HS) as opposed to the Rotorod Test.

**TABLE I**

| Comparative Median Effective Dosage | | | |
|---|---|---|---|
| Compound | Tox TD50 mg/kg | MES ED50 mg/kg | sc Met ED50 mg/kg |
| D,L-α-acetamido-N-benzyl-3-thiopheneacetamide | >100 | 87.80 | ≠ |
| D,L-α-acetamido-N-benzyl-2-thiopheneacetamide | 30-100 | 44.80 | ≠ |
| D,L-α-acetamido-N-benzyl-2-furanacetamide | 40 | 10.33 | ≠ |
| D,L-α-acetamido-N-benzyl-2-pyrroleacetamide | <100 | 16.10 | ≠ |
| D,L-2-acetamido-N-benzyl-2-ethoxyacetamide | >112 | 62.01 | ≠ |
| D,L-2-acetamido-N-benzyl-2-methoxyacetamide | <300 | 98.30 | ≠ |
| phenytoin | 66 | 10 | not effective |
| phenobarbital | 69 | 22 | 13 |
| mephenytoin | 154 | 61 | 31 |
| phenacemide | 421 (337-549)* | 87 (74-100)* | 116 (71-150)* |

| | | | |
|---|---|---|---|
| * 95% confidence intervals | | | |
| ≠ The ED50 for this substrate was not computed | | | |

## Claims (Claims for the following Contracting State(s): BE, CH, DE, FR, GB, IT, LI, LU, NL, SE)

1. An anticonvulsant composition comprising an anticonvulsant effective amount of a compound having the following general formula: 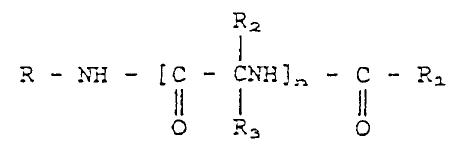 wherein R is aryl, aryl lower alkyl, heterocyclic, lower alkyl heterocyclic, polynuclear aromatic or lower alkyl polynuclear aromatic, each unsubstituted or substituted with at least one electron withdrawing substituent or at least one electron donating substituent;
R₁ is H or a lower alkyl, unsubstituted or substituted with at least one electron withdrawing substituent or at least one electron donating substituent;
R₂ and R₃, independently, are hydrogen, lower alkenyl, lower alkynyl, heterocyclic, lower alkyl heterocyclic, polynuclear aromatic, lower alkyl polynuclear aromatic, each unsubstituted or substituted with at least one electron withdrawing substituent or at least one electron donating substituent, or R₂ and R₃ can be a halogen or a heteroatom containing oxygen, nitrogen, sulfur, or phosphorous substituted with hydrogen, lower alkyl or aryl, said lower alkyl or aryl groups being substituted or unsubstituted; with the proviso that R₂ and R₃ are not both hydrogen;
n is 1 to 4;
wherein lower alkyl substituents have from 1 to 6 carbon atoms; aryl substituents have from 6 to 10 ring carbon atoms; heterocyclic substituents have up to 4 heteroatoms and up to 13 ring carbon atoms; polynuclear aromatic compounds have up to 4 fused rings and up to 18 ring carbon atoms; lower alkenyl and lower alkynyl substituents containing from 2 to 6 carbon atoms; and
wherein electron withdrawing groups can be halo, nitro, acyl, carboxyl, carboalkoxy, carboxamide, cyano, sulfonyl, sulfoxide, heterocyclic, guanidine or quaternary ammonium; and electron donating groups can be hydroxy, alkoxy, lower alkyl, amino, phenoxy, thiol, sulfide or disulfide and
a pharmaceutically acceptable carrier.

2. The composition according to Claim 1 wherein R₁ is hydrogen, methyl, ethyl, propyl, isopropyl, butyl, isobutyl, tertiary butyl, amyl or hexyl.

3. The composition in accordance with Claims 1 and 2 wherein said methyl, ethyl, propyl, isopropyl, butyl, isobutyl, tertiary butyl, amyl or hexyl are unsubstituted or substituted with at least one electron withdrawing substituent or at least one electron donating substituent as defined in claim 1.

4. The composition in accordance with Claims 1-3 wherein R is benzyl, unsubstituted or substituted with at least one electron withdrawing substituent or at least one electron donating substituent as defined in claim 1.

5. The composition according to Claim 1 wherein R₂ or R₃ is lower alkoxy.

6. The composition in accordance to Claim 1
wherein R is benzyl, R₁ is methyl, R₂ is (3-thienyl), R₃ is hydrogen and n is 1;
R is benzyl, R₁ is methyl, R₂ is (2-thienyl), R₃ is hydrogen and n is 1;
R is benzyl, R₁ is methyl, R₂ is (2-furyl), R₃ is hydrogen and n is 1;
R is benzyl, R₁ is methyl, R₂ is (2-Pyrrolyl), R₃ is hydrogen and n is 1;
R is benzyl, R₁ is methyl, R₂ is ethoxy, R₃ is hydrogen and n is 1; or
R is benzyl, R₁ is methyl, R₂ is methoxy, R₃ is hydrogen and n is 1.

7. The composition in accordance with Claims 1-7 wherein said compound is in the D isomer form, L isomer form, mixtures of the D,L isomer form or D,L racemate form.

8. The composition according to Claim 1 wherein the compound is:
D,L-α-acetamido-N-benzyl-3-thiophenacetamide;
D,L-α-acetamido-N-benzyl-2-thiophenacetamide;
D,L-α-acetamido-N-benzyl-2-furanacetamide;
D,L-α-acetamido-N-benzyl-2-pyrrolacetamide;
D,L-2-acetamido-N-benzyl-2- ethoxyacetamide;
D,L-2-acetamido-N-benzyl-2-methoxyacetamide.

9. Use of a compound of formula: 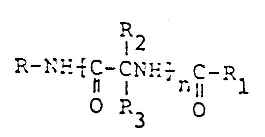 where R, R₁, R₂, R₃ and n are as defined in claim 1, or a salt thereof in the preparation of an anticonvulsant medicament.

## Claims (Claims for the following Contracting State(s): ES, AT, GR)

1. A process for producing an anticonvulsant composition, characterized in that an
anticonvulsant effective amount of a compound having the following general formula: 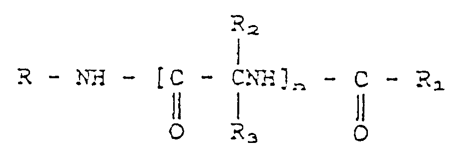 wherein R is aryl, aryl lower alkyl, heterocyclic, lower alkyl heterocyclic, polynuclear aromatic or lower alkyl polynuclear aromatic, each unsubstituted or substituted with at least one electron withdrawing substituent or at least one electron donating substituent;
R₁ is H or a lower alkyl, unsubstituted or substituted with at least one electron withdrawing substituent or at least one electron donating substituent;
R₂ and R₃, independently, are hydrogen, lower alkenyl, lower alkynyl, heterocyclic, lower alkyl heterocyclic, polynuclear aromatic, lower alkyl polynuclear aromatic, each unsubstituted or substituted with at least one electron withdrawing substituent or at least one electron donating substituent, or R₂ and R₃ can be a halogen or a heteroatom containing oxygen, nitrogen, sulfur, or phosphorous substituted with hydrogen, lower alkyl or aryl, said lower alkyl or aryl groups being substituted or unsubstituted; with the proviso that R₂ and R₃ are not both hydrogen;
n is 1 to 4;
wherein lower alkyl substituents have from 1 to 6 carbon atoms; aryl substituents have from 6 to 10 ring carbon atoms; heterocyclic substituents have up to 4 heteroatoms and up to 13 ring carbon atoms; polynuclear aromatic compounds have up to 4 fused rings and up to 18 ring carbon atoms; lower alkenyl and lower alkynyl substituents containing from 2 to 6 carbon atoms; and
wherein electron withdrawing groups can be halo, nitro, acyl, carboxyl, carboalkoxy, carboxamide, cyano, sulfonyl, sulfoxide, heterocyclic, guanidine or quaternary ammonium; and electron donating groups can be hydroxy, alkoxy, lower alkyl, amino, phenoxy, thiol, sulfide or disulfide is formulated with a pharmaceutically acceptable carrier.

2. A process according to Claim 1 wherein R₁ is hydrogen, methyl, ethyl, propyl, isopropyl, butyl, isobutyl, tertiary butyl, amyl or hexyl.

3. A process according to Claims 1 and 2 wherein said methyl, ethyl, propyl, isopropyl, butyl, isobutyl, tertiary butyl, amyl or hexyl are unsubstituted or substituted with at least one electron withdrawing substituent or at least one electron donating substituent as defined in claim 1.

4. A process according to Claims 1-3 wherein R is benzyl, unsubstituted or substituted with at least one electron withdrawing substituent or at least one electron donating substituent as defined in claim 1.

5. A process according to Claim 1 wherein R₂ or R₃ is lower alkoxy.

6. A process according to Claim 1
wherein R is benzyl, R₁ is methyl, R₂ is (3-thienyl), R₃ is hydrogen and n is 1;
R is benzyl, R₁ is methyl, R₂ is (2-thienyl), R₃ is hydrogen and n is 1;
R is benzyl, R₁ is methyl, R₂ is (2-furyl), R₃ is hydrogen and n is 1;
R is benzyl, R₁ is methyl, R₂ is (2-pyrrolyl), R₃ is hydrogen and n is 1;
R is benzyl, R₁ is methyl, R₂ is ethoxy, R₃ is hydrogen and n is 1, or
R is benzyl, R₁ is methyl, R₂ is methoxy, R₃ is hydrogen and n is 1.

7. A process according to Claims 1-7 therein said compound is in the D isomer form, L isomer form, mixtures of the D,L isomer form or D,L racemate form.

8. A process according to Claim 1 wherein the compound is:
D,L-α-acetamido-N-benzyl-3-thiophenacetamide;
D,L-α-acetamido-N-benzyl-2-thiophenacetamide;
D,L-α-acetamido-N-benzyl-2-furanacetamide;
D,L-α-acetamido-N-benzyl-2-pyrrolacetamide;
D,L-2-acetamido-N-benzyl-2- ethoxyacetamide;
D,L-2-acetamido-N-benzyl-2-methoxyacetamide.

9. Use of a compound of formula: 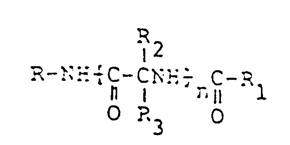 where R, R₁, R₂, R₃ and n are as defined in claim 1, or a salt thereof in the preparation of an anticonvulsant medicament.

## Patentansprüche (Patentansprüche für folgende(n) Vertragsstaat(en): BE, CH, DE, FR, GB, IT, LI, LU, NL, SE)

1. Krampfhemmende Zusammensetzung, die eine für die Krampfhemmung wirksame Menge einer Verbindung mit der folgenden allgemeinen Formel 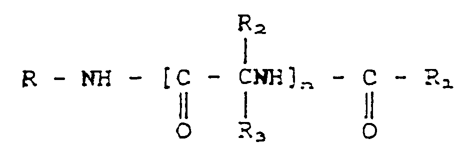 wobei R eine Arylgruppe, Arylniedrigalkylgruppe, heterozyklische Gruppe, niedrigalkylheterozyklische Gruppe, vielkernige aromatische Gruppe oder niedrigalkyl-vielkernig aromatische Gruppe ist, die jeweils unsubstituiert sind oder mit mindestens einem elektronenziehenden oder mindestens einem elektronenspendenden Substituenten substituiert sind;
wobei R₁ Wasserstoff oder eine Niedrigalkylgruppe ist, die unsubstituiert ist oder mit mindestens einem elektronenziehenden Substituenten oder mindestens einem elektronenspendenden Substituenten substituiert ist;
wobei R₂ und R₃ unabhängig voneinander Wasserstoff, eine Niedrigalkenylgruppe, eine Niedrigalkynylgruppe, eine heterozyklische Gruppe, eine niedrigalkyl-heterozyklische Gruppe, eine vielkernig aromatische Gruppe, eine niedrigalkyl-vielkernig aromatische Gruppe sind, die jeweils unsubstituiert sind oder mit mindestens einem elektronenziehenden oder mindestens einem elektronenspendenden Substituenten substituiert sind, oder R₂ und R₃ ein Halogenatom oder ein Sauerstoff, Stickstoff, Schwefel oder Phosphor enthaltendes Heteroatom, substituiert mit Wasserstoff, einer Niedrigalkyl- oder -arylgruppe, wobei die Niedrigalkyl- oder -arylgruppen substituiert oder unsubstituiert sind, sein können; unter der Voraussetzung, daß R₂ und R₃ nicht gleichzeitig Wasserstoffatome sind;
wobei n eine Zahl von 1 bis 4 ist;
wobei die Niedrigalkylsubstituenten 1 bis 6 Kohlenstoffatome aufweisen, die Arylsubstituenten 6 bis 10 Ringkohlenstoffatome besitzen; die heterozyklischen Substituenten bis zu 4 Heteroatome und bis zu 13 Ringkohlenstoffatome aufweisen; die vielkernig aromatischen Verbindung bis zu 4 verschmolzene Ringe und bis zu 18 Ringkohlenstoffatome besitzen; die Niedrigalkenyl- und Niedrigalkynylsustituenten 2 bis 6 Kohlenstoffatome aufweisen; und
wobei die elektronenziehenden Gruppen Halo, Nitro, Acyl, Carboxyl, Carbalkoxy, Carboxamid, Cyano, Sulfonyl, Sulfoxid, eine heterozyklische Gruppe, Guanidin oder quaternäres Ammonium sein können; und die elektronenspendenden Gruppen Hydroxy, Alkoxy, eine Niedrigalkylgruppe, Amino, Phenoxy, Thiol, Sulfid oder Disulfid sein können;
und einen pharmazeutisch verträglichen Träger umfaßt.

2. Zusammensetzung nach Anspruch 1, wobei R₁ Wasserstoff, Methyl, Ethyl, Propyl, Isopropyl, Butyl, Isobutyl, tert.-Butyl, Amyl oder Hexyl ist.

3. Zusammensetzung nach Anspruch 1 und 2, wobei die Methyl-, Ethyl-, Propyl-, Isopropyl-, Butyl-, Isobutyl-, tert.-Butyl-, Amyl- oder Hexylgruppe unsubstituiert ist oder mit mindestens einem elektronenziehenden Substituenten oder mit mindestens einem elektronenspendenden Substituenten, wie sie in Anspruch 1 definiert sind, substituiert ist.

4. Zusammensetzung nach den Ansprüchen 1 bis 3, wobei R Benzyl ist, das unsubstituiert ist oder mit mindestens einem elektronenziehenden Substituenten oder mindestens einem elektronenspendenden Substituenten gemäß der Definition in Anspruch 1 substituiert ist.

5. Zusammensetzung nach Anspruch 1, wobei R₂ oder R₃ eine Niedrigalkoxygruppe ist.

6. Zusammensetzung nach Anspruch 1, wobei R Benzyl, R₁ Methyl, R₂ (3-Thienyl), R₃ Wasserstoff und n 1 ist;
R Benzyl, R₁ Methyl, R₂ (2-Thienyl), R₃ Wasserstoff und n 1 ist;
R Benzyl, R₁ Methyl, R₂ (2-Furyl), R₃ Wasserstoff und n 1 ist;
R Benzyl, R₁ Methyl, R₂ (2-Pyrrolyl), R₃ Wasserstoff und n 1 ist;
R Benzyl, R₁ Methyl, R₂ Ethoxy, R₃ Wasserstoff und n 1 ist; oder
R Benzyl, R₁ Methyl, R₂ Methoxy, R₃ Wasserstoff und n 1 ist.

7. Zusammensetzung nach den Ansprüchen 1 bis 7, wobei die Verbindung in der isomeren D-Form, der isomeren L-Form, als Mischungen der isomeren D- und L-Formen oder als D,L-Razemat vorliegt.

8. Zusammensetzung nach Anspruch 1, wobei die Verbindung dargestellt ist durch:
D,L-alpha-Acetamido-N-benzyl-3-thiophenacetamid;
D,L-alpha-Acetamido-N-benzyl-2-thiophenacetamid;
D,L-alpha-Acetamido-N-benzyl-2-furanacetamid;
D,L-alpha-Acetamido-N-benzyl-2-pyrrolacetamid;
D,L-2-Acetamido-N-benzyl-2-ethoxyacetamid;
D,L-2-Acetamido-N-benzyl-2-methoxyacetamid.

9. Verwendung einer Verbindung der Formel 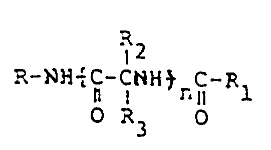 wobei R, R₁, R₂, R₃ und n wie in Anspruch 1 definiert sind, oder eines Salzes hiervon zur Herstellung eines krampfhemmenden Arzneimittels.

## Patentansprüche (Patentansprüche für folgende(n) Vertragsstaat(en): ES, AT, GR)

1. Verfahren zur Herstellung einer krampfhemmenden Zusammensetzung, dadurch gekennzeichnet, daß eine für die Krampfhemmung wirksame Menge einer Verbindung der folgenden allgemeinen Formel 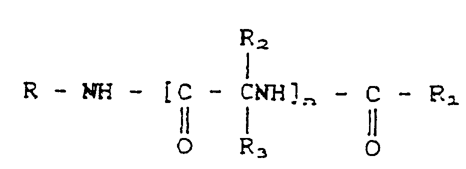 wobei R eine Arylgruppe, Arylniedrigalkylgruppe, heterozyklische Gruppe, niedrigalkylheterozyklische Gruppe, vielkernige aromatische Gruppe oder niedrigalkyl-vielkernig aromatische Gruppe ist, die jeweils unsubstituiert sind oder mit mindestens einem elektronenziehenden oder mindestens einem elektronenspendenden Substituenten substituiert sind;
wobei R₁ Wasserstoff oder eine Niedrigalkylgruppe ist, die unsubstituiert ist oder mit mindestens einem elektronenziehenden Substituenten oder mindestens einem elektronenspendenden Substituenten substituiert ist;
wobei R₂ und R₃ unabhängig voneinander Wasserstoff, eine Niedrigalkenylgruppe, eine Niedrigalkynylgruppe, eine heterozyklische Gruppe, eine niedrigalkyl-heterozyklische Gruppe, eine vielkernig aromatische Gruppe, eine niedrigalkyl-vielkernig aromatische Gruppe sind, die jeweils unsubstituiert sind oder mit mindestens einem elektronenziehenden oder mindestens einem elektronenspendenden Substituenten substituiert sind, oder R₂ und R₃ ein Halogenatom oder ein Sauerstoff, Stickstoff, Schwefel oder Phosphor enthaltendes Heteroatom, substituiert mit Wasserstoff, einer Niedrigalkyl- oder -arylgruppe, wobei die Niedrigalkyl- oder -arylgruppen substituiert oder unsubstituiert sind, sein können; unter der Voraussetzung, daß R₂ und R₃ nicht gleichzeitig Wasserstoffatome sind;
wobei n eine Zahl von 1 bis 4 ist;
wobei die Niedrigalkylsubstituenten 1 bis 6 Kohlenstoffatome aufweisen, die Arylsubstituenten 6 bis 10 Ringkohlenstoffatome besitzen; die heterozyklischen Substituenten bis zu 4 Heteroatome und bis zu 13 Ringkohlenstoffatome aufweisen; die vielkernig aromatischen Verbindung bis zu 4 verschmolzene Ringe und bis zu 18 Ringkohlenstoffatome besitzen; die Niedrigalkenyl- und Niedrigalkynylsustituenten 2 bis 6 Kohlenstoffatome aufweisen; und
wobei die elektronenziehenden Gruppen Halo, Nitro, Acyl, Carboxyl, Carbalkoxy, Carboxamid, Cyano, Sulfonyl, Sulfoxid, eine heterozyklische Gruppe, Guanidin oder quaternäres Ammonium sein können; und die elektronenspendenden Gruppen Hydroxy, Alkoxy, eine Niedrigalkylgruppe, Amino, Phenoxy, Thiol, Sulfid oder Disulfid sein können, mit einem pharmazeutisch verträglichen Träger formuliert wird.

2. Verfahren nach Anspruch 1, wobei R₁ Wasserstoff, Methyl, Ethyl, Propyl, Isopropyl, Butyl, Isobutyl, tert.-Butyl, Amyl oder Hexyl ist.

3. Verfahren nach Anspruch 1 und 2, wobei die Methyl-, Ethyl-, Propyl-, Isopropyl-, Butyl-, Isobutyl-, tert.-Butyl-, Amyl- oder Hexylgruppe unsubstituiert ist oder mit mindestens einem elektronenziehenden Substituenten oder mit mindestens einem elektronenspendenden Substituenten, wie sie in Anspruch 1 definiert sind, substituiert ist.

4. Verfahren nach Ansprüchen 1 bis 3, wobei R Benzyl ist, das unsubstituiert ist oder mit mindestens einem elektronenziehenden Substituenten oder mindestens einem elektronenspendenden Substituenten gemäß der Definition in Anspruch 1 substituiert ist.

5. Verfahren nach Anspruch 1, wobei R₂ oder R₃ eine Niedrigalkoxygruppe ist.

6. Verfahren nach Anspruch 1, wobei R Benzyl, R₁ Methyl, R₂ (3-Thienyl), R₃ Wasserstoff und n 1 ist;
R Benzyl, R₁ Methyl, R₂ (2-Thienyl), R₃ Wasserstoff und n 1 ist;
R Benzyl, R₁ Methyl, R₂ (2-Furyl), R₃ Wasserstoff und n 1 ist;
R Benzyl, R₁ Methyl, R₂ (2-Pyrrolyl), R₃ Wasserstoff und n 1 ist;
R Benzyl, R₁ Methyl, R₂ Ethoxy, R₃ Wasserstoff und n 1 ist; oder
R Benzyl, R₁ Methyl, R₂ Methoxy, R₃ Wasserstoff und n 1 ist.

7. Verfahren nach den Ansprüchen 1 bis 7, wobei die Verbindung in der isomeren D-Form, der isomeren L-Form, als Mischungen der isomeren D- und L-Formen oder als D,L-Razemat vorliegt.

8. Verfahren nach Anspruch 1, wobei die Verbindung dargestellt ist durch:
D,L-alpha-Acetamido-N-benzyl-3-thiophenacetamid;
D,L-alpha-Acetamido-N-benzyl-2-thiophenacetamid;
D,L-alpha-Acetamido-N-benzyl-2-furanacetamid;
D,L-alpha-Acetamido-N-benzyl-2-pyrrolacetamid;
D,L-2-Acetamido-N-benzyl-2-ethoxyacetamid;
D,L-2-Acetamido-N-benzyl-2-methoxyacetamid.

9. Verwendung einer Verbindung der Formel 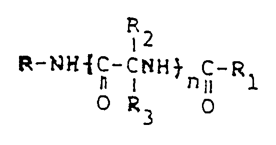 wobei R, R₁, R₂, R₃ und n wie in Anspruch 1 definiert sind, oder eines Salzes hiervon zur Herstellung eines krampfhemmenden Arzneimittels.

## Revendications (Revendications pour l'(les) Etat(s) contractant(s) suivant(s): BE, CH, DE, FR, GB, IT, LI, LU, NL, SE)

1. Composition anticonvulsivante comprenant une quantité efficace anticonvulsivante d'un composé ayant la formule générale suivante: 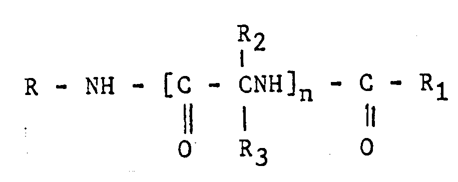 dans laquelle R représente un aryle, un alkyle inférieur d'aryle, un hétérocycle, un hétérocycle d'alkyle inférieur, un aromatique polynucléaire ou un aromatique polynucléaire à alkyle inférieur , chacun non-substitué ou substitué par au moins un substituant attracteur d'électrons ou au moins un substituant donneur d'électrons;
R₁ représente un hydrogène ou un alkyle inférieur, non-substitué ou substitué par au moins un substituant attracteur d'électrons ou au moins un substituant donneur d'électrons;
R₂ et R₃ représentent, indépendemment, un hydrogène, un alkényle inférieur, un alkynyle inférieur, un hétérocycle, un hétérocycle d'alkyle inférieur, un aromatique polynucléaire, un aromatique polynucléaire à alkyle inférieur, chacun étant non-substitué ou substitué par au moins un substituant attracteur d'électrons ou au moins un substituant donneur d'électrons, ou R₂ et R₃ peuvent représenter un halogène ou un hétéroatome contenant de l'oxygène, de l'azote, du soufre, ou un phosphore substitué par de l'hydrogène, un aryle ou un alkyle inférieur, lesdits groupes aryle ou alkyle inférieur étant non-substitués ou substitués; à la condition que R₂ et R₃ ne représentent pas tous les deux des hydrogènes;
n est compris entre 1 et 4;
dans laquelle les substituants alkyles inférieurs ont de 1 à 6 atomes de carbone; les substituants aryles ont de 6 à 10 atomes de carbone cycliques; les substituants hétérocycliques ont jusqu'à 4 hétéroatomes et jusqu'à 13 atomes de carbone cycliques; les composés aromatiques polynucléaires ont jusqu'à 4 cycles condensés et jusqu'à 18 atomes de carbone cycliques; les substituants alkynyles inférieurs et alkényles inférieurs contenant de 2 à 6 atomes de carbone; et
dans laquelle les groupes attracteurs d'électrons peuvent être halo, nitro, acyle, carboxyle, carboalcoxy, carboxamide, cyano, sulfonyle, sulfoxyde, héterocyclique, guanidine ou ammonium quaternaire; et les groupes donneurs d'électrons peuvent être hydroxy, alcoxy, alkyle inférieur, amino, phénoxy, thiol, sulfure ou disulfure et
un support pharmaceutiquement compatible.

2. Composition selon la revendication 1 dans laquelle R₁ représente hydrogène, méthyle, éthyle, propyle, isopropyle, butyle, isobutyle, t-butyle, amyle ou hexyle.

3. Composition selon les revendications 1 et 2 dans laquelle lesdits méthyle, éthyle, propyle, isopropyle, butyle, isobutyle, t-butyle, amyle ou hexyle sont non-substitués ou substitués par au moins un substituant attracteur d'électrons ou par au moins un substituant donneur d'électrons tel que défini dans la revendication 1.

4. Composition selon les revendications 1-3 dans laquelle R représente un benzyle, non-substitué ou substitué par au moins un substituant attracteur d'électrons ou par au moins un substituant donneur d'électrons tel que défini dans la revendication 1.

5. Composition selon la revendication 1 dans laquelle R₂ ou R₃ représente un alcoxy inférieur.

6. Composition selon la revendication 1 dans laquelle R est benzyle, R₁ est méthyle, R₂ est (3-thiényle), R₃ est l'hydrogène et n est 1;
R est benzyle, R₁ est méthyle, R₂ est (2-thiényle), R₃ est l'hydrogène et n est 1;
R est benzyle, R₁ est méthyle, R₂ est (2-furyle), R₃ est l'hydrogène et n est 1;
R est benzyle, R₁ est méthyle, R₂ est (2-pyrrolyle), R₃ est l'hydrogène et n est 1;
R est benzyle, R₁ est méthyle, R₂ est éthoxy, R₃ est l'hydrogène et n est 1; ou
R est benzyle, R₁ est méthyle, R₂ est méthoxy, R₃ est l'hydrogène et n est 1.

7. Composition conformément aux revendications 1 à 7 dans laquelle ledit composé est sous la forme de l'isomère D , sous la forme de l'isomère L, de mélanges des formes d'isomères D et L ou d'une forme racémique D,L.

8. Composition selon la revendication 1 dans laquelle le composé est :
D,L-α-acétamido-N-benzyl-3-thiophènacétamide;
D,L-α-acétamido-N-benzyl-2-thiophènacétamide;
D,L-α-acétamido-N-benzyl-2-furanacétamide;
D,L-α-acétamido-N-benzyl-2-pyrrolacétamide;
D,L-2-acétamido-N-benzyl-2-éthoxyacétamide;
D,L-2-acétamido-N-benzyl-2-méthoxyacétamide.

9. Utilisation d'un composé de formule: 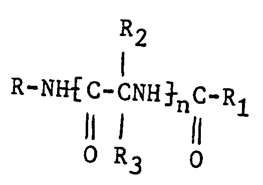 dans laquelle R, R₁, R₂, R₃ et n sont tels que définis dans la revendication 1, ou un sel de ceux-ci dans la préparation d'un médicament anticonvulsivant.

## Revendications (Revendications pour l'(les) Etat(s) contractant(s) suivant(s): ES, AT, GR)

1. Procédé pour la préparation d'une composition anticonvulsivants, caractérisé en ce qu'une quantité efficace anticonvulsivante d'un composé ayant la formule générale suivante: 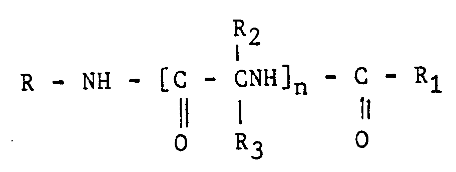 dans laquelle R représente un aryle, un alkyle inférieur d'aryle, un hétérocycle, un hétérocycle d'alkyle inférieur, un aromatique polynucléaire ou un aromatique polynucléaire à alkyle inférieur , chacun non-substitué ou substitué par au moins un substituant attracteur d'électrons ou au moins un substituant donneur d'électrons;
R₁ représente un hydrogène ou un alkyle inférieur, non-substitué ou substitué par au moins un substituant attracteur d'électrons ou au moins un substituant donneur d'électrons;
R₂ et R₃ représentent, indépendemment, un hydrogène, un alkényle inférieur, un alkynyle inférieur, un hétérocycle, un hétérocycle d'alkyle inférieur, un aromatique polynucléaire, un aromatique polynucléaire à alkyle inférieur, chacun étant non-substitué ou substitué par au moins un substituant attracteur d'électrons ou au moins un substituant donneur d'électrons, ou R₂ et R₃ peuvent représenter un halogène ou un hétéroatome contenant de l'oxygène, de l'azote, du soufre, ou un phosphore substitué par de l'hydrogène, un aryle ou un alkyle inférieur, lesdits groupes aryle ou alkyle inférieur étant substitués ou non-substitués; à la condition que R₂ et R₃ ne représentent pas tous les deux des hydrogènes;
n est compris entre 1 et 4;
dans laquelle les substituants alkyles inférieurs ont de 1 à 6 atomes de carbone; les substituants aryles ont de 6 à 10 atomes de carbone cycliques; les substituants hétérocycliques ont jusqu'à 4 hétéroatomes et jusqu'à 13 atomes de carbone cycliques; les composés aromatiques polynucléaires ont jusqu'à 4 cycles condensés et jusqu'à 18 atomes de carbone cycliques; les substituants alkynyles inférieurs et alkényles inférieurs contenant de 2 à 6 atomes de carbone; et
dans laquelle les groupes attracteurs d'électrons peuvent être halo, nitro, acyle, carboxyle, carboalcoxy, carboxamide, cyano, sulfonyle, sulfoxyde, hétérocyclique, guanidine ou ammonium quaternaire; et les groupes donneurs d'électrons peuvent être hydroxy, alcoxy, alkyle inférieur, amino, phénoxy, thiol, sulfure ou disulfure
est mise en forme galénique avec un support pharmaceutiquement compatible.

2. Procédé selon la revendication 1 dans lequel R₁ représente hydrogène, méthyle, éthyle, propyle, isopropyle, butyle, isobutyle, t-butyle, amyle ou hexyle.

3. Procédé selon les revendications 1 et 2 dans lequel lesdits méthyle, éthyle, propyle, isopropyle, butyle, isobutyle, t-butyle, amyle ou hexyle sont non-substitués ou substitués par au moins un substituant attracteur d'électrons ou par au moins un substituant donneur d'électrons tel que défini dans la revendication 1.

4. Procédé selon les revendications 1-3 dans lequel R représente un benzyle, non-substitué ou substitué par au moins un substituant attracteur d'électrons ou par au moins un substituant donneur d'électrons tel que défini dans la revendication 1.

5. Procédé selon la revendication 1 dans lequel R₂ ou R₃ représente un alcoxy inférieur.

6. Procédé selon la revendication 1 dans lequel R est benzyle, R₁ est méthyle, R₂ est (3-thiényle), R₃ est l'hydrogène et n est 1;
R est benzyle, R₁ est méthyle, R₂ est (2-thiényle), R₃ est l'hydrogène et n est 1;
R est benzyle, R₁ est méthyle, R₂ est (2-furyle), R₃ est l'hydrogène et n est 1;
R est benzyle, R₁ est méthyle, R₂ est (2-pyrrolyle), R₃ est l'hydrogène et n est 1;
R est benzyle, R₁ est méthyle, R₂ est éthoxy, R₃ est l'hydrogène et n est 1; ou
R est benzyle, R₁ est méthyle, R₂ est méthoxy, R₃ est l'hydrogène et n est 1.

7. Procédé selon les revendications 1 à 7 dans lequel ledit composé est sous la forme de l'isomère D , sous la forme de l'isomère L, de mélanges des formes d'isomères D et L ou d'une forme racémique D,L.

8. Procédé selon la revendication 1 dans lequel le composé est :
D,L-α-acétamido-N-benzyl-3-thiophènacétamide;
D,L-α-acétamido-N-benzyl-2-thiophènacétamide;
D,L-α-acétamido-N-benzyl-2-furanacétamide;
D,L-α-acétamido-N-benzyl-2-pyrrolacétamide;
D,L-2-acétamido-N-benzyl-2-éthoxyacétamide;
D,L-2-acétamido-N-benzyl-2-méthoxyacétamide.

9. Utilisation d'un composé de formule: 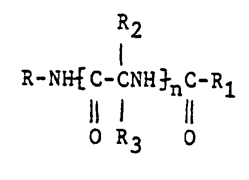 dans laquelle R, R₁, R₃, R₃ et n sont tels que définis dans la revendication 1, ou un sel de ceux-ci dans la préparation d'un médicament anticonvulsivant.
